# DEMANDE DE BREVET EUROPEEN

(11) **EP 0 581 643 A1**
(43) Date de publication de la demande: **02.02.1994**
(21) Numéro de dépôt: 93401877.1
(22) Date de dépôt: 21.07.1993
(51) Int. Cl.: G01N 33/24, G01V 3/26, G01V 3/40, G01N 27/72

(54) **Procédé de datation d'une couche géologique**

(30) Priorité: 30.07.1992 FR 9209435
(71) Demandeur: COMPAGNIE GENERALE DE GEOPHYSIQUE, F-91341 Massy Cédex (FR)
(72) Inventeur: Martin, Jean-Pierre, F-92700 Colombes (FR)
(74) Mandataire: Flavenot, Bernard

(57) **Abrégé**

La présente invention concerne les procédés de datation des différents niveaux d'une couche géologique terrestre.

Le procédé selon l'invention consiste à mesurer l'aimantation rémanente le long d'un puits foré dans la couche géologique, à détecter l'ensemble des inversions de l'aimantation rémanente, à mesurer l'aimantation induite aux différents niveaux de cette couche, à détecter les maxima d'aimantation induite et à établir une corrélation entre ces différentes mesures et les dates connues, d'une part des inversions du champ magnétique terrestre et d'autre part de phénomènes terrestres ayant entraîné des concentrations de produits fortement magnétiques, pour attribuer une date aux différents niveaux de la couche.

Application, notamment, à la recherche pétrolière.

## Description

La présente invention concerne les procédés de datation des différents niveaux d'une couche géologique terrestre, qui trouvent des applications particulièrement avantageuses dans le domaine de la prospection et de l'exploitation pétrolières.

Afin de faciliter la recherche des gisements de pétrole, la détermination de leur qualité et leur mise en exploitation optimale, il est très avantageux de pouvoir dater les différents niveaux des couches géologiques dans lesquelles sont entreprises les prospections et l'exploitation des gisements.

Différents procédés sont connus pour effectuer une telle datation, notamment ceux qui consistent à relever les valeurs de l'aimantation rémanente dans les différents niveaux des couches géologiques prospectées.

En effet, avant d'aller plus loin dans la présente description, il est nécessaire de rappeler que, sur la Terre, règne un champ magnétique qui est actuellement orienté vers le point dénommé "nord", mais que, au cours des ères géologiques passées, il a basculé un grand nombre de fois entre ce point "nord" et le point "sud". On sait en outre que les roches terrestres soumises au champ magnétique terrestre possèdent une aimantation qui comprend deux composantes, l'une représentant l'aimantation induite par le champ magnétique terrestre actuel, que les techniciens dénomment "aimantation induite", l'autre représentant l'aimantation dite "rémanente" correspondant à l'aimantation induite par le champ magnétique terrestre qui régnait au moment de la formation de ces roches et dont elles ont mémorisé la direction et le sens.

Ainsi, pour dater les différents niveaux d'une couche géologique, on commence généralement par forer un puits, avantageusement perpendiculairement au sol, puis on mesure, au moyen d'un appareil connu en lui-même, par exemple celui qui est décrit dans le Brevet FR-A-2 652 911, l'aimantation rémanente tout le long du puits, de façon à relever l'altitude des points pour lesquels il y a une inversion du sens de cette aimantation.

Comme on connaît, dans l'échelle des temps, ceux qui correspondent aux inversions de l'orientation du champ magnétique terrestre, il est alors théoriquement facile de dater chaque niveau de couche compris entre deux points correspondant à deux inversions successives du champ magnétique terrestre au cours du temps.

Le procédé succinctement décrit ci-dessus donne de bons résultats, à condition que les couches géologiques analysées aient eu une formation homogène, par simples dépôts et sédimentation, sans l'intervention de phénomènes et/ou catastrophes, par exemple des basculements, des failles, des discontinuités par érosion, etc. En effet, dans ce cas, les strates des couches géologiques sont tellement perturbés que l'interprétation des résultats des mesures de l'aimantation rémanente le long d'un puits peut conduire à des incertitudes, lacunes ou, dans le pire des cas, des erreurs de datation

La présente invention a ainsi pour but de mettre en oeuvre un procédé de datation des différents niveaux d'une couche géologique terrestre, qui permet d'éliminer un certain nombre de lacunes et/ou d'incertitudes inhérentes aux procédés connus de l'art antérieur.

Plus précisément, la présente invention a pour objet un procédé de datation des différents niveaux d'une couche géologique terrestre, caractérisé par le fait qu'il consiste:
- à mesurer l'aimantation rémanente en des points situés à différents niveaux de ladite couche géologique,
- à détecter, dans l'ensemble desdites mesures de l'aimantation rémanente, celles correspondant à une inversion du sens de ladite aimantation rémanente et à délivrer un premier signal représentatif de l'ensemble des inversions de l'aimantation rémanente en fonction desdits niveaux,
- à mesurer l'aimantation induite en des points situés à différents niveaux de ladite couche géologique,
- à détecter, dans l'ensemble desdites mesures de l'aimantation induite, celles correspondant à des maxima de ladite aimantation induite et à délivrer un deuxième signal représentatif de l'ensemble desdits maxima d'aimantation induite en fonction desdits niveaux, et
- à établir une corrélation entre lesdits premier et deuxième signaux et les dates connues, d'une part des inversions du champ magnétique terrestre et d'autre part de phénomènes terrestres ayant entraîné des concentrations de produits fortement magnétiques, pour attribuer une date aux différents niveaux de ladite couche.

D'autres caractéristiques et avantages de la présente invention apparaîtront au cours de la description suivante donnée en regard des dessins annexés à titre illustratif, mais nullement limitatif, dans lesquels:
la figure 1 représente un exemple de coupe d'une couche géologique, qui permet d'expliciter la mise en oeuvre du procédé selon l'invention,
La figure 2 représente une courbe illustrant un signal représentatif de la variation de l'aimantation rémanente à différents niveaux "h" de la couche géologique selon la figure 1,
La figure 3 représente une courbe illustrant un signal représentatif des maxima de l'aimantation induite à différents niveaux "h" de la couche géologique selon la figure 1, et
La figure 4 représente une courbe de variation du champ magnétique terrestre en fonction du temps géologique "t" et, en superposition, une courbe situant, dans la même échelle de temps géologique, des phénomènes terrestres ayant entraîné des concentrations de produits fortement magnétiques, comme les éruptions volcaniques.

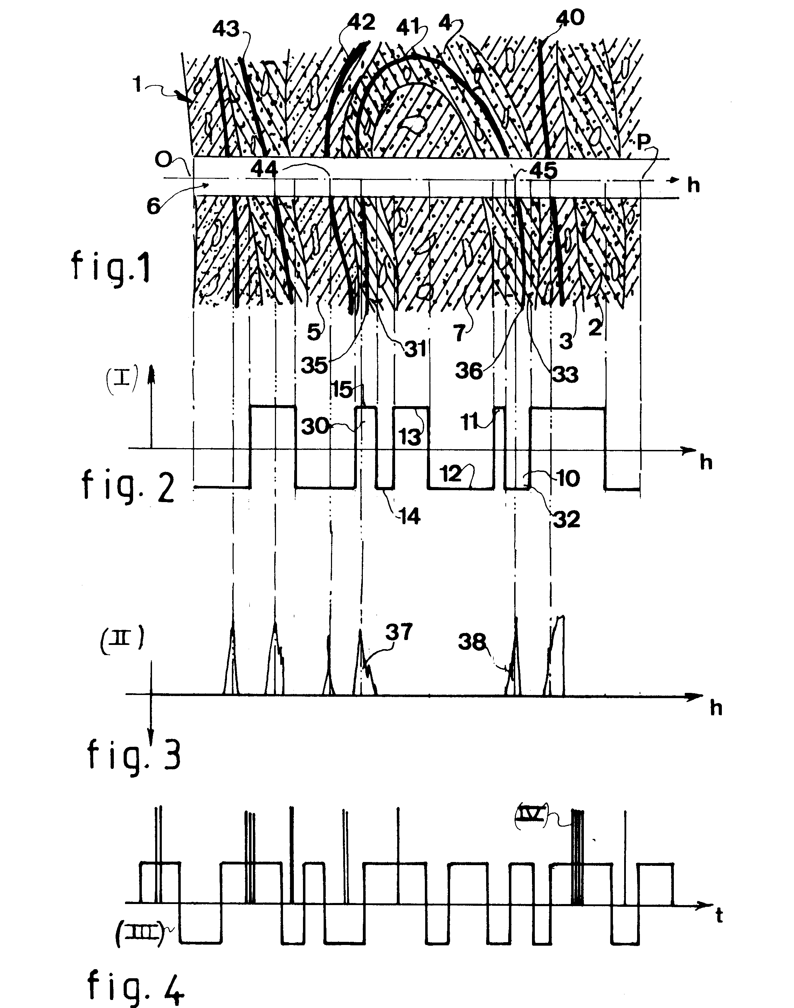

Il est tout d'abord précisé que les différentes représentations graphiques données sur les figures 1 à 4, aussi bien de la couche géologique elle-même que des différents signaux définis ci-dessus, sont tout à fait fictives et ne sont données qu'à titre d'exemple illustratif permettant d'expliciter le procédé selon l'invention.

Afin de faciliter la recherche des gisements de pétrole, la détermination de leur qualité et leur mise en exploitation optimale, il est très avantageux de pouvoir dater les différents niveaux des couches géologiques dans lesquelles sont entreprises les prospections et l'exploitation des gisements.

La figure 1 représente un exemple de coupe d'une couche géologique 1 dont il est nécessaire de dater les différents niveaux entre les points "O" et "P", notamment les strates 2, 3, 4, 5, 7, ... pour en déduire l'époque de leur formation et, par exemple, évaluer la probabilité de la présence d'hydrocrabures et savoir s'il faut poursuivre une recherche pétrolière ou non.

Pour effectuer la datation des différents niveaux de la couche 1, on commence généralement par forer un puits 6, avantageusement verticalement.

Puis on descend dans ce puits un dispositif connu en lui-même apte à mesurer la variation de l'aimantation rémanente mesurée le long du puits, par exemple entre les deux points "O" et "P".

Dans l'ensemble de ces mesures, on détecte les variations de l'aimantation rémanente correspondant à une inversion du sens de cette aimantation rémanente et on identifie les niveaux, entre les deux points "O" et "P" de la couche géologique, pour lesquels l'aimantation rémanente est inversée. Il est alors élaboré un premier signal représentatif de l'ensemble de toutes les inversions de l'aimantation rémanente en fonction de l'altitude "h" des niveaux entre les points "O" et "P" de la couche géologique 1. La courbe 1 représentée sur la figure 2 illustre ce premier signal déduit des mesures de l'aimantation rémanente le long du puits 6 entre les point "O" et "P".

Il faut en effet rappeler que, sur la Terre, règne un champ magnétique qui est actuellement orienté vers le point dénommé "nord", mais que, au cours des temps géologiques passés, ce champ magnétique terrestre a basculé un grand nombre de fois entre ce point "nord" et le point "sud". Ces inversions sont bien connues en elles-mêmes et parfaitement datées dans l'échelle des temps. On sait en outre que les roches terrestres qui ont été soumises au champ magnétique terrestre possèdent une aimantation dite "rémanente" correspondant à l'aimantation induite par le champ magnétique terrestre qui régnait au moment de leur formation et mémorisée par elles. L'une des deux courbes représentées sur la figure 4, la courbe III, donne un exemple fictif des variations de l'aimantation rémanente au cours du temps géologique "t", dans lesquelles se remarquent facilement les inversions de cette aimantation rémanente.

Si la formation de la couche géologique entre les deux points "O" et "P" a été homogène, c'est-à-dire si les différents strates définis entre ces deux points se sont déposés les uns sur les autres et n'ont pas subi de bouleversements notables au cours du temps géologique, en corrélant les courbes 1 et III, il est possible de dater, dans la couche géologique 1 prospectée, les niveaux où se sont produites les inversions de l'aimantation terrestre.

Par contre, si les strates ont subi des bouleversements et/ou des catastrophes, tels que des plissements, basculements, etc, ils peuvent prendre une allure comme celle qui est représentée à titre illustratif sur la figure 1. Dans la courbe géologique 1, le strate identifié par la référence 4 s'est replié en emprisonnant le strate 7 et le signal délivré par le dispositif de mesure détecte donc une orientation de l'aimantation rémanente 30 pour la partie haute 31 du strate 4 et une orientation 32 de sens contraire pour la partie basse 33 de ce même strate 4. La courbe 1 représentant le premier signal met donc en évidence un nombre d'inversions de l'aimantation rémanente, par exemple les inversions 10, 11, 12, 13, 14, 15, supérieur celui qui s'est produiten réalité. En conséquence, le premier signal est erroné et il devient impossible d'établir une corrélation entre les deux courbes 1 et III, et donc de dater tous les différents niveaux entre les points "O" et "P".

Il existe donc dans ce cas une incertitude ou une lacune dans la datation des différents niveaux de la couche géologique 1.

Le procédé selon l'invention permet, dans de nombreux cas, de lever ces incertitudes ou lacunes.

Il est en effet connu que, au cours des ères géologiques, se sont produits des phénomènes terrestres ayant entraîné des concentrations importante de produits fortement magnétiques, notamment de la magnétite. Parmi ces phénomènes, on peut citer de très fortes éruptions volcaniques qui se sont fait ressentir sur de vastes étendues de la Terre, ou d'importantes évolutions glaciaires, comme des avancées ou des reculs de glaciers, qui ont concentré sur de grandes surfaces, notamment, des produits fortement magnétiques. Ces phénomènes sont dans leur grande majorité répertoriés et identifiés, notamment par leur durée, la quantité et la nature des produits éjectés ou rassemblés. La courbe IV de la figure 4, tracée avec la même échelle de temps géologique "t" que la courbe III, donne un exemple fictif permettant de situer dans le temps des éruptions volcaniques connues de différente importance.

Il est en outre connu que les produits émis lors des éruptions volcaniques contiennent des corps fortement magnétiques, par exemple celui qui est dénommé "magnétite", dont l'une des propriétés est de donner à ces produits une aimantation induite beaucoup plus forte que celle des roches ordinaires contenues dans les couches géologiques. Les strates provenant de dépôts consécutifs aux retombées des produits éjectés lors d'éruptions volcaniques ont donc une aimantation induite nettement plus forte que les strates qui ne contiennent pas de tels produits.

C'est ainsi que, pour la mise en oeuvre du procédé selon l'invention, par exemple au moyen du même dispositif mentionné au préambule de la présente description, on mesure l'aimantation induite le long du puits 6 en ne retenant dans ces mesures, par exemple par filtrage, que celles correspondant aux valeurs dites "maxima" de l'aimantation induite, c'est-à-dire celles qui dépassent un certain seuil prédéterminé. L'ensemble de ces portions de mesures filtrées constitue un deuxième signal généralement composé de pics, comme représenté par la courbe Il de la figure 3 en fonction de l'altitude "h" des différents niveaux de la couche géologique 1. Ce deuxième signal permet donc de déterminer les niveaux de la couche géologique 1 où règne une aimantation induite nettement plus intense qu'en leur aval ou leur amont, c'est-à-dire les niveaux où se situent, dans la couche géologique, des dépôts consécutifs à des retombées de produits éruptifs volcaniques fortement magnétiques, chaque pic correspondant sensiblement, sur la figure 1, à un trait continu épais 40,41,42 et 43 représentant un tel depôt.

En effectuant une corrélation entre la courbe Il représentant le deuxième signal et la courbe IV représentant l'ensemble des éruptions volcaniques répertoriées dans le temps, il est possible de lever certaines incertitudes ou de combler certaines lacunes dans la datation des différents niveaux d'une couche géologique.

Par exemple, en se reportant à la couche géologique 1 illustrée sur la figure 1, on va commencer par effectuer une première datation selon la méthode connue antérieurement, en effectuant une corrélation entre la courbe avec la courbe III. La corrélation entre la courbe Il et la courbe IV permet de dater avec certitude certains niveaux de la couche 1, puisque les éruptions volcaniques sont en général parfaitement identifiables les unes par rapport aux autres, ce qui n'est pas le cas des inversions de l'aimantation rémanente.

Dans l'exemple illustré, on va pouvoir de plus déterminer que l'une des deux portions 37, 38 du deuxième signal de la courbe II, par exemple la portion 38, correspond à une éruption volcanique répertoriée, tandis que l'autre, la portion 37, ne correspond a priori à aucune éruption. Cependant, comme ces deux portions sont symétriques l'une de l'autre dans une symétrie axiale, il existe une grande probabilité pour que ces deux portions 37, 38 de la courbe Il soient représentatives de l'aimantation induite d'un même dépôt de produits éruptifs volcaniques. On peut ainsi en déduire que les portions de strates 31 et 33 qui contiennent respectivement les portions 35, 36 de dépôts de produits éruptifs volcaniques appartiennent à un même strate qui s'est replié, comme le strate 4 dans l'exemple illustré.

L'incertitude qui pouvait exister dans la datation des niveaux entre les deux points 44 et 45 de la couche 1, appartenant respectivement aux deux portions de strates 31 et 33, est donc levée par l'analyse selon le procédé ci-dessus.

L'exemple donné ci-dessus en se référant aux éruptions volcaniques peut s'appliquer de la même façon aux évolutions glaciaires dont la plupart sont aussi parfaitement répertoriées.

## Revendications

1. Procédé de datation des différents niveaux d'une couche géologique terrestre, caractérisé par le fait qu'il consiste:
- à mesurer l'aimantation rémanente en des points situés à différents niveaux de ladite couche géologique,
- à détecter, dans l'ensemble desdites mesures de l'aimantation rémanente, celles correspondant à une inversion du sens de ladite aimantation rémanente et à délivrer un premier signal représentatif de l'ensemble des inversions de l'aimantation rémanente en fonction desdits niveaux,
- à mesurer l'aimantation induite en des points situés à différents niveaux de ladite couche géologique,
- à détecter, dans l'ensemble desdites mesures de l'aimantation induite, celles correspondant à des maxima de ladite aimantation induite et à délivrer un deuxième signal représentatif de l'ensemble desdits maxima d'aimantation induite en fonction desdits niveaux, et
- à établir une corrélation entre lesdits premier et deuxième signaux et les dates connues, d'une part des inversions du champ magnétique terrestre et d'autre part de phénomènes terrestres ayant entraîné des concentrations de produits fortement magnétiques, pour attribuer une date aux différents niveaux de ladite couche géologique.

2. Procédé selon la revendication 1, caractérisé par le fait que les phénomènes terrestres ayant entraîné des concentrations de produits fortement magnétiques sont des éruptions volcaniques.

3. Procédé selon la revendication 1, caractérisé par le fait que les phénomènes terrestres ayant entraîné des concentrations de produits fortement magnétiques sont des évolutions glaciaires.
